# EUROPÄISCHE PATENTSCHRIFT

(11) **EP 0 379 891 B1**
(45) Veröffentlichungstag und Bekanntmachung des Hinweises auf die Patenterteilung: **08.06.1994**
(21) Anmeldenummer: 90100472.1
(22) Anmeldetag: 10.01.1990
(51) Int. Cl.: F26B 25/00, B41F 23/04, G01N 21/35

(54) **Durchlauftrockner von Rollenrotationsdruckmaschinen und sein Betriebsverfahren**
Continuous dryer for web-fed rotary presses and its operating process
Séchoir pour presses à bobines et son procédé de fonctionnement

(30) Priorität: 25.01.1989 DE 3902180; 27.11.1989 DE 3939190
(43) Veröffentlichungstag der Anmeldung: 01.08.1990
(73) Patentinhaber: Baldwin-Gegenheimer GmbH, D-86068 Augsburg (DE)
(72) Erfinder: Waizmann, Franz, D-8901 Gessertshausen (DE)
(74) Vertreter: Vetter, Ewald Otto, Dipl.-Ing.

(56) Entgegenhaltungen:
- EP-A- 0 094 706
- EP-A- 0 123 458
- EP-A- 0 346 041
- DE-A- 2 150 259
- US-A- 3 905 126
- US-A- 3 909 954
- US-A- 4 343 096

## Beschreibung

Die Erfindung betrifft einen Durchlauftrockner von Rollenrotationsdruckmaschinen nach dem Oberbegriff des Anspruches 1 und sein Betriebsverfahren.

Ein solcher Durchlauftrockner findet bei Drucklinien Anwendung, wo eine Bahn von der Rolle abgewickelt, in Druckwerken passergenau mit Bildern bedruckt, durch einen thermisch wirkenden Durchlauftrockner zu einem Falzapparat zur Exemplarisierung abgewickelt wird.

In den "Sicherheitsregeln für Explosionsschutz an Durchlauftrocknern von Druck- und Papierverarbeitungsmaschinen", Carl Heymanns Verlag KG, Bestell-Nr. ZH1/19, Oktober 1984 sind Merkmale der Durchlauftrockner und ihres Betriebs beschrieben. Danach werden in einen Durchlauftrockner Lösemittel oder lösemittelhaltige Stoffe wie Druckfarben usw. eingebracht, die mit Luft explosionsfähige Lösemitteldampf-Luftgemische bilden können. Aufgrund der angeordneten ungeschützten Heizungen, mit denen die zum Trockenkanal geführte, erhitzte Frisch- und Umluft auf Temperatur gebracht wird, besteht zwischen Trockenkanal und Heizraum keine gasdichte Trennung. Vom Hersteller oder Betreiber der Durchlauftrockner ist eine Grenztemperatur festzulegen, die unterhalb der Zündtemperatur, siehe DIN 51794 und unterhalb der Verbrennungserscheinungen ergebenden Entzündungstemperatur zu liegen hat. Die ungeschützte Heizung ist dadurch definiert, daß die Temperatur des erhitzten Luftstroms auch partiell die Grenztemperatur überschreitet. Die Grenztemperatur ist auf 80 % der nach DIN 51794 gemessenen Zündtemperatur vereinbart.
Den brennbaren Stoffen ist eine untere Explosionsgrenze (UEG) zugeordnet, die sich nach dem unteren Grenzwert der Konzentration von Gasen und Dämpfen, bei der sich nach dem Zünden eine von der Zündquelle unabhängige Flamme gerade nicht mehr selbstündig fortpflanzen kann, bemißt. Bei Durchlauftrocknern mit ungeschützter Heizung im Umluftkanal wird von einer Nichtüberschreitung der Lösemitteldampf-Konzentration unmittelbar vor der ungeschützten Heizung von 25 % der UEG ausgegangen. Als Anhaltswert für die UEG der Lösemittel bei nicht bekannter Lösemittel-Dampf Konzentration dient der Wert von 20 g/m³.

Wegen der beim Trocknerbetrieb einzuhaltenden Grenztemperatur muß die Heizung des Durchlauftrockners mit einem Temperaturregler, zumindest mit einer Temperaturbegrenzung versehen sein. Weiterhin ist es durch die Vorschrift bekannt, die Begrenzung der Lösemittel-Dampf-Konzentration im Durchlauftrockner von einer Gaswarneinrichtung zu überwachen, die die explosionsfähige Atmosphäre in gefahrdrohender Menge sicher erfassen soll. Der Meßgrößenaufnehmer der Gaswarneinrichtung soll mindestens 5 Meßwerte pro Minute und Meßstelle liefern.

Es besteht das Problem, daß die vorschriftsmäßig einzubauenden Gaswarneinrichtungen lediglich in Zeitabständen und von einer sogenannten repräsentativen Stelle aus Meßwerte liefern, womit plötzlichem Konzentrationswechsel oder stofflichem Wechsel der mit der Bahn eingeschleusten, verdampfenden Substanzen nicht Rechnung getragen werden kann. Außerdem leitet sich die Prozeßführung des Trockners nicht von der effektiven Gas- bzw. Dampfkonzentration ab, sondern folgt dem bei ungeschützten Heizungen an der Entzündungstemperatur orientierten Temperaturprogramm.

Zur Überwachung der im Durchlauftrockner entstehenden Lösemitteldampf-Konzentration ist es üblich, einen FID (Flammen-Ionisations-Detektor) einzusetzen. Aus der DE-B-2150259 ist ein an einen Absaugkamin eines Tiefdrucktrockners angeschlossenes Gasmeßgerät bekannt, mit dem die Gasanteile von Toluol in der Luft feststellbar sind.

Bekannte IR-Meßgeräte können je nach Einsatzzweck sehr verschiedenartigen Aufbau haben. Der Gegenstand nach der EP-A-0123458 weist für das zu messende Gas eine kleine, zu beschickende Probenkammer auf. Zur Erzeugung von Strahlung im mit Absorption behafteten Wellenlängenbereich sind Filter vorgesehen. Beim Gegenstand nach der DE-A-2211835 sind für die Absorptionsbanden der zu analysierenden Gaskomponenten Interferenzfilter eingesetzt. Bei der Vorgabe von vornherein nur auf den Absorptionsbereich spektral beschränkter Strahlung mit Hilfe eines Lasers ist der Aufbau nur für die eine Komponente brauchbar oder nur mit erheblichem Aufwand für einen breiteren, spektralen Strahlenbereich durchstimmbar. Ein Absorptionsaufbau mit einem Laser ist in der US-A-4475816 aufgezeigt.

Die Gegenstände mit Meßkammer und Probennahme weisen den Nachteil auf, daß die Gaskomponentenmessung vom Wert her nicht repräsentativ und außerdem zu langsam erfolgt. Bei der FID-Messung eilen die Meßwerte nach. Die bekannten IR-Meßaufbauten stützen sich zwar auf jeweils bekannte Raukomponenten und physikalische Zusammenhänge, die eine Vielzahl von Meßaufgaben zu lösen gestatten, doch sind die für die Betriebsmessungen bekannten IR-Meßeinrichtungen auf ungeeignetes Meßkopfprinzip ausgerichtet, während andere bekannte IR-Meßeinrichtungen lediglich Laborcharakter aufweisen.

Es stellt sich daher bei dem Durchlauftrockner der bekannten Gattung und bei einem Betrieb eines derartigen Trockners die Aufgabe, die Trocknung unter Einbeziehung der Brennerkontrolle nach den Konzentrationswerten der abdampfenden Komponenten zu führen, wobei der Feststellung der Konzentrationswerte schnelles Ansprechen und gleich gute Anwendbarkeit für jede der vorkommenden Komponenten anhaften soll.

Die Lösung dieser Aufgabe besteht aus den in Anspruch 1 angegebenen Merkmalen. Weitere Lösungen hinsichtlich des Betriebs sind durch die Merkmale der Ansprüche 2-4 dargestellt.

Folgende Vorteile sind mit der Lösung verbunden:
Die Gas/Dampfmessung über IR-Absorption arbeitet ohne zusätzliche Gasversorgung wie beim FID. Die für die Absorptionsmessung zu bildende Strahlstrecke ist einfach aufzubauen, weil es nur auf die Durchstrahlung der gas/dampfhaltigen Atmosphäre an irgendeiner dafür geeigneten Stelle im Durchlauftrockner ankommt. Geeignet sind daher die apparativ vorliegenden Strömungswege bzw. -kanäle. Die Strahlstrecke läßt sich auch in der Nähe der Heizung, bei ungeschützter Heizung des Gasbrennrtyps in der Nähe der mit heißen Verbrennungsgasen angereicherten Umluft anordnen. Die von den Brenn- und Abgasen verursachte Absorption läßt sich für die Brennerführung nutzen. Auch die Flamme ist durchstrahlbar.

Die Strahlstrecke ist weitgehend temperaturunempfindlich. Möglicherweise kondensierende, spiegel- oder linsenverschmutzende Anteile, die den Strahlengang oder die Strahlenintensität verfälschen würden, sind durch Aufheizung auf eine Temperatur oberhalb des Kondensationspunktes freizuhalten. Andererseits können Verschmutzungen automatisch oder von Hand von Zeit zu Zeit beseitigt werden.

Die Intensität der Strahlung kann einfach durch Temperaturerhöhung, d.h. Leistungserhöhung, gesteigert werden, um den Detektionserfordernissen zu genügen.

Zur Feststellung der aus der Druckfarbe und aus der Waschflüssigkeit beim Gummituchwaschen ausdampfenden Flüssiganteile kann zunächst mittels IR-Spektrometer eine grundsätzliche Identification der maßgeblichen Komponenten (qualitative Analyse, Konstitutionsanalyse) vorgenommen werden. Das Infrarot-Spektrometer arbeitet in einem Fall zur Vermessung eines breiten Spektrums, nämlich demjenigen des Grundschwingungsgebiets, mit der Fourier-Transformation. Nach Feststehen der wesentlichen Komponenten kann der Empfang auf diese Komponenten beschränkt werden, wozu für die Absorptionsbereiche dieser Komponenten entweder entsprechende Filter in den Strahlengang gelegt oder selektiv arbeitende Detektoren angeordnet werden. Damit sind gezielt z.B. die zum Verfestigen der Druckfarbe beigegebenen, austreibbaren Öle sowie Harze und z.B. das im Reinigungsmittel für das Gummituchwaschen enthaltene Waschbenzin in Art und Menge feststellbar. Bei einem einfachen IR-Meßaufbau sind entsprechend der zu vermessenden Komponenten entsprechend viele engbandige "Fester" angeordnet, wobei die Fenster auch durch spezifisch empfangende Detektoren darstellbar sind.

Grundsätzlich sind für die Strahlführung Spiegel oder Linsen anwendbar. Spiegel, Planspiegel oder sphärische Spiegel zur konvergierenden Bündelung des Lichts sind einfacher als Linsen und z.B. aus Metall. Zur Aufteilung eines Strahls in einen weiterlaufenden Strahlteil und einen abgelenkten Strahlteil kann z.B. ein halbdurchlässiger Spiegel angewendet werden. Die Strahlführung kann auch auf bewegliche Filter, Spiegel, Schirme abgestellt werden.

Zur quantitativen Auswertung wird die durch absorption geschwächt erscheinende Intensität der charakteristischen Banden der jeweiligen Substanz mit einer benachbart liegenden, relativ konstanten, geschwächten oder ungeschwächten Intensität verglichen. Die Netto-Maximalextinktion ergibt sich aus dem logarithmierten Verhältnis bzw. der Differenzbildung der logarithmierten Werte von festgelegtem Basiswert bei relativ konstanter oder maximaler Durchlässigkeit zum Wert der Spitze der Schlüsselbande.
Weil die Bandentiefe bei der Absorptionswellenlänge logarithmisch mit der Konzentration und der Strahlweglänge zunimmt, ist Signalverstärkung und bessere Abhebung der Asorptionsbanden gegenüber dem Untergrund mittels Strahlwegverlängerung erreichbar, wofür der Strahlweg wie beim mehrfachen Gasküvettendurchgang auch faltbar ist.

Zum Zurückwerfen des Strahls im Hinblick darauf, daß Strahlungsquelle und Strahlungsempfänger nicht entgegengesetzt angeordnet sind, ist ein in seiner mechanischen Justierung relativ unempfindlicher Retroreflektor, ein sogenanntes Katzenauge, einsetzbar. Der Strahlengang verläuft durch ihn minimal parallel versetzt zurück.

Grundsätzlich lassen sich die optischen Elemente zum Aufbau der IR-Strahlenstrecke innerhalb des Durchlauftrockners anordnen. Dabei sind sie sowohl der verschmutzenden, dampfhaltigen Atmosphäre ausgesetzt, als auch Temperaturgang und Wärmeverzug- und Spannung unterworfen, worunter die präzise Strahlführung leiden kann. Nach Auswahl des zur Messung gesählten, infraroten Spektralbereichs - vorzugsweise mittleres Infrarot MIR der Wellenzahl 4000 bis 400 - sind geeignete Fenster anzubringen, mit denen der heiße Trockner raumdicht, jedoch optisch durchlässig insofern baulich veränderbar ist, daß empfindliche optische Elemente zugänglich und stabil außerhalb montierbar sind. Die breitbandig durchlässigen Fenster sind zugänglich angeflanscht. Gegen Kondensatverschmutzung und Strömungsschmutzfahnen sind die Fenster im jeweils geeigneten, relativ gleichmäßige Strömung und Erwärmung aufweisenden Teil des Trockners anzuordnen. Die zu justierenden optischen Elemente sind von stabilen Trägern gehalten. Neben der IR-Strahlungsquelle kann noch eine andere, denselben Justierungsbedingungen unterworfene Lichtquelle angeordnet sein, deren Strahlengang auf eine Empfängermatrix fällt, die Abweichungen vom Soll-Strahlungsverlauf signalisiert und aufgrund der Abweichungen eine automatische Nachführung der optischen Elemente hin zur Soll-Lage betätigt. Die IR-Meßstrecke kann auch über den IR-Meßstrahl selbst mit einer Justierung versehen sein.

Die Druckfarben, die ungefähr 10 bis 30 % Pigmente eingebettet in Bindemittel des Anteils 60 bis 70 % enthalten, ergeben ein kennzeichnendes IR-Spektrum, das von den Mineralölen, pflanzlichen Ölen und Harzen stammt. Die Rezeptur der Druckfarben durch den Herstellprozeß und seitens der verschiedenartigen Anforderungen für die Druckfarben ist nicht gleich. Die im Trockner nennenswert verdampfenden Bindemittelstoffe hinterlassen typische, teilweise identifizierbare, katalogisierte Banden bzw. Bandensätze, teilweise nicht oder schwer aufklärbare Absorptionsverläufe. Unabhängig davon ist das jeweilige druckfarbenzugehörige Spektrogramm spezifisch nach Bandenlage und auch Bandenintensität festliegend. Änderungen der stofflichen Zusammensetzung der Druckfarbe mit der Wirkung von z.B. Verdruckbarkeitsstörungen (Feuchtmittelaufnahme, Wegschlagen ins Papier, Ablegen bei schlechter Trockenbarkeit, Aufbauen usw.) können aus Einzelheiten des Spektrums ersehen werden. Aus der Bandenintensität, damit aus dem Konzentrationswert eines Stoffes der Druckfarbe in der Dampfphase kann wertmäßig auf den Anteil und auf die Abdampfkinetik geschlossen werden, so daß sich anhand der spektralen Werte interpretierbare und sogar analysierbare Informationen über Bindemittelzusammensetzung und partielle Substanzverdampfung ergeben.

Ein bestimmtes, typisches Spektrogramm bezüglich einer Druckfarbe kann mit einem anderen Spektrogramm bezüglich derselben, angeblichen Druckfarbe oder einer anderen Druckfarbe verglichen werden, wobei die dem Strahlungsempfänger nachgeschaltete Auswerteeinheit beispielsweise autokorrelative Methoden anwendet.

Für den zur Heißlufterzeugung betriebenen Verbrennungsprozeß ist die Verbrennung dadurch kontrollierbar, daß die an sich restlos verbrennenden Brenngase auf Vorhandensein und die Verbrennung selbst durch unvollkommen verbrannte Produkte untersucht werden. Im Brenngas befinden sich über IR-Absorption nachweisbare gasförmige Kohlenwasserstoffe. Ihr Vorhandensein in der Trockneratmosphäre erfordert verbesserte Führung der Verbrennung. Nachweisbare CO-Anteile zeigen ebenfalls, daß die stöchiometrische Verbrennung nicht erreicht ist. Die gasförmigen Kohlenwasserstoffverbindungen des Brenngases weisen in den aufgenommenen Spektren bestimmte Banden auf, mit denen Anhaltspunkte über die Verbrennung geliefert werden und die Verbrennung quantifizierbar ist. Beispielsweise ergibt Methan CH₄ als kleines Molekül einen eindeutigen Bandensatz, mit dem bei der Gehaltsbestimmung vorgegangen werden kann.

Eine stoff- bzw. bandenspezifische Auswertung kann stattfinden, wenn gegenüber einem vorgegebenen Stoff analysiert wird, der entweder praktisch in der Meßanordnung als Vergleichsprobe eingerichtet wird oder rechnerisch bezüglich seiner Bande bzw. Bandensatzes berücksichtigt wird. Bei der Vorgabe der Vergleichsprobe kann eine abgesperrte Küvette mit dem Vergleichsgas oder dem Vergleichsgasgemisch in den Strahlengang gelegt werden. Für Vergleichsmessungen ist auch ein dem Strahlengang angepaßtes Strömungsrohr möglich, durch welches Vergleichsgas, Inertgas oder Tracer-Gas bekannter Zusammensetzung geschickt wird. Die Vergleichsgasbeladung des Trockners ist in ihrem Einfluß bei den hohen Stoffdurchsätzen vernachlässigbar.

Die Anwendung eines Vergleichsgases kann sich aus mehreren Gründen als zweckmäßig erweisen. Bei Verwendung absorptionsfreier Gase, z.B. Stickstoff, kann die Grundintensität gemessen werden oder durch Beimischung eingemessener Mengen des spezifischen Gases/Dampfes zum absorptionsfreien Gas eine Meßreihe aufgestellt werden.

Die Abzielung auf die eine oder die mehreren wichtigen Komponenten der im IR Spektrum insgesamt wiedergegebenen Stoffe der Trockneratmosphäre ist aufgrund des normalerweise vielgestaltigen Meßspektrums nur durch Auswertung der betreffenden charakteristischen Banden möglich. Diese sind im einfachsten Fall aus Tabellen entnehmbar, andererseits können die entscheidenden, auf die Druckfarben, die Reinigungsflüssigkeit sowie Brenn- und Abgas entfallenden Substanzen für sich durch folgende Methoden behandelt werden:
- Die Substanz wird für sich dargestellt und in einer Meßreihe eine Eichkurve zwischen Extinktion und Konzentration erstellt;
- die Substanz mit bekanntem Konzentrationswert wird während der Betriebsmessung in den Meßstrahl eingeschaltet, wo sie eine ständige, definierte Absorption hervorruft. Thre quantitative Berücksichtigung erfolgt gemäß einer bekannten, nicht-dispersiven Methode mit "Meßgasfilter";
- für die Substanz wird ein selektiv in Höhe der betreffenden Bande bzw. des Bandensatzes arbeitendes Filter vorgesehen.

Natürlich kann auch ein Meßverlauf dadurch geschaffen werden, daß Probenmengen der betreffenden Substanz auf der Papierbahn in den Trockner eingefahren werden. Dabei kann gleichzeitig das Übergangsverhalten der Substanz in Abhängigkeit der Parameter des als offenes System anzusehenden Durchlauftrockners und in Abhängigkeit der weiteren Einflußgrößen wie Bahngeschwindigkeit, Papierart usw. aufgenommen werden.

Im wenig veränderlichen, stationären Betrieb können bei angeordneten FID-Meßstellen von dort kommende Gesamtkohlenwasserstoff-Meßwerte mit den jeweiligen Bandenintensitäten verglichen werden.

Aufgenommene On-Line-Meßwerte der IR-Absorptionsmessung werden zweckmäßig gespeichert, damit der jeweils herrschende Betriebszustand bezüglich Druckfarbe, flüssigem Reinigungsmittel für die Gummitücher und Brenner mit Vergleichsdaten oder Soll-Daten gegenübergestellt werden kann.

Während die Durchlauftrockner in Bezug auf einige Betriebseinrichtungen bisher nur für stationären Betrieb mit festen Einstellungen betrieben werden können, um Gefahrenmomente aufgrund willkürlicher Veränderungen auszuschließen, ist der Trocknerbetrieb mit Hilfe der Gas/Dampfkonzentrationsfeststellung und damit die Kontrolle der entzündbaren Atmosphäre nunmehr vielseitiger gestaltbar.

Bei mehreren Trocknersektionen mit eingeteilter Trocknungswirkung von Zone zu Zone in Ablaufrichtung der Bahn sind die Konzentrationen von Zone zu Zone meßbar und der Trocknungsverlauf mittels Eingriff auf die Temperatur- und Strömungsverhältnisse gezielt einstellbar.
Querempfindlichkeit bei Kohlenwasserstoffen mit Sauerstoffbindung, bei CO, CO₂, die den Einsatz des FID einschränkt, herrscht bei der IR-Absorptionsmessung nicht.
Der Strahlengang ist mittel einschwenkbarer oder einschiebbarer, optisch wirksamer Elemente wie Spiegel, Filter, Küvetten, vielseitig den Anforderungen anpaßbar. Mit den Wasser zurechenbaren Banden, die schon vom Wasserdampf normaler Umgebungsluft stammen und zusätzlich auf verdampftes Wasser des Papiers sowie des Feuchtmittels einschließlich des Wasserdampfes des verbrannten Brenngases zurückgehen, ist insofern eine Wasserbilanz aufstellbar, als die erscheinenden Intensitäten je nach Prozeßführung Untershiede zeigen. Die Prozeßführung ist in Bahnlauf ohne/mit Druck, Langsam/Schnellbahnlauf, geringe und starke Flächendeckung der Bedruckung, niedrige/hohe Trockentemperatur, niedrige/hohe Brenngas-, Zu-, Ab-, Umluftdurchsätze unterschiedbar.
Die IR-Strahlung ist wegen der vielen im Trockner angeordneten Oberflächen mit Absorptions/Reflexionseigenschaft teilweise diffus und verlustbehaftet. Außerdem zeigt das IR-Spektrometer apparative Quereinflüsse, so daß Meßverläufe auf die jeweils effektive Nullintensität zu beziehen sind. Die Nullintensität ist abzufragen oder verlaufsweise, intervallweise festzulegen.
Bei Auslauf des Fortdrucks bzw. Anlauf des Gummituchwaschens sowie Wiederanfahren des Fortdrucks bzw. Auslauf des Reinigungsvorganges ergeben sich jeweils zurückgehende bzw. ansteigende Bandenintensitäten entsprechend der Druckfarbe und Reinigungsflüssigkeit zugehörigen Kohlenwasserstoffkomponenten.

Im folgenden wird die Erfindung anhand eines Ausführungsbeispiels dargestellt.

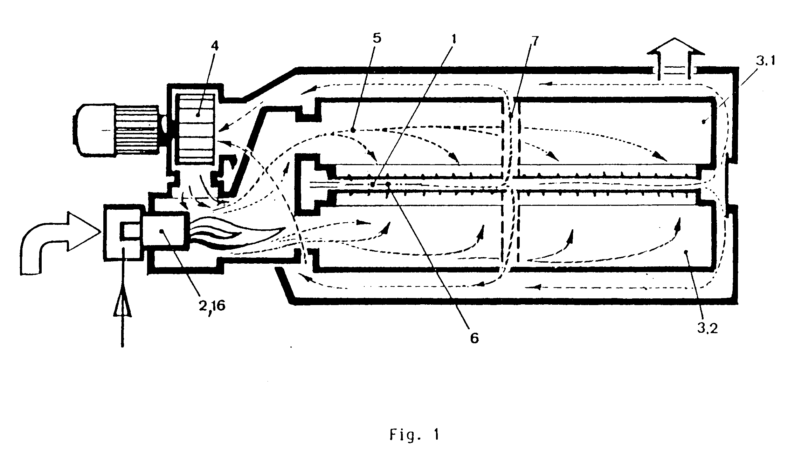

Es zeigen:
- Fig. 1:: ein Durchlauftrockner schematisiert im Querschnitt;
- Fig. 2A:: ein Durchlauftrockner schematisiert räumlich;
- Fig. 2B:: geometrischer Raumkörper des Trockners mit IR-Strahleinrichtungen und Strahlengang;
- Fig. 3:: ein Durchlauftrockner schematisiert im Längsschnitt dargestellt;
- Fig. 4A:: schematisierte Darstellung eines FTIR-Spektrometers;
- Fig. 4B:: vereinfachter Strahlengang für eine nicht dispersiv abtastende Strahlungsanordnung;
- Fig. 5:: Durchstrahlung der Apparatewand des Durchlauftrockners
a) Strahl mit Retroreflexion durch Fenster in einem Rohr;
b) Durchstrahlung mit Strahlablenkung durch Fenster zweier gegenüberliegender Wände;
c) Durchstrahlung mit Retroreflexion mit ortsfest montierlem Retroreflektor;
- Fig. 6A:: ein IR-Spektrum, Abszisse Wellenzahl linear, Ordinate Durchlässigkeit, Grundschwingungssgebiet MlR, für einen beliebigen Kohlenwasserstoff;
- Fig. 6B:: Auswertung der Bandenintensität nach dem Basislinienverfahren;
Nach Fig. 1 besteht der Durchlauftrockner für eine bedruckte Bahn 1 mit bedruckter Schöndruck- und Widerdruckseite aus einer Heizeinrichtung 2 als Lufterhitzer, einem oberen Düsenkasten 3.1, einem unteren Düsenkasten 3.2, einer Umluftströmungseinrichtung 4. Zuluft- und Abluftstrom sind durch Stoffstrompfeile angegeben. Die Umluftströmung im Trockner verläuft nach den vereinfacht wiedergegebenen Stromlinienpfeilen 5 vorerhitzt durch die Düsenkästen 3.1, 3.2 zum Bahnkanal 6, wo sie auf die Schöndruck- und Widerdruckseite der laufenden Bahn trifft, und gelangt rückströmend angereichert mit von der Bahn 1 verdampften Anteilen der Druckfarbe oder der Reinigungsflüssigkeit zurück zur Heizungseinrichtung 2. Die stationäre Gas/Dampfkonzentration im Durchlauftrockner wird über die Zu- und Abluft reguliert, weil der Farbmengenstrom je nach Druckfläche und Farbdeckung sowie Bahngeschwindigkeit variiert. Zur Regulierung von Zu- und Abluft sind die zugehörigen Strömungsmaschinen 17, 18 regelbar und zusätzlich Strömungsklappen betätigbar.

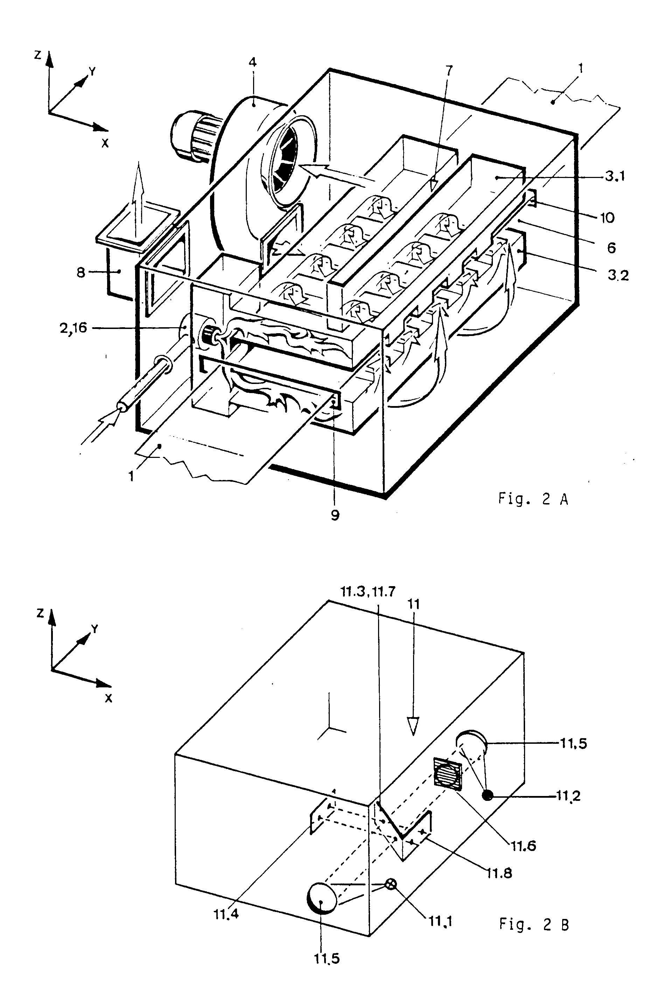

Die Strömungs- und damit Gas/Dampfkonzentrationsvergleichmäßigung ist wesentlich von der Gestaltung der Strömungswege abhängig. Zur Verkürzung der Rückströmwege und damit Verringerung des Strömungsverlustes weisen die Düsenkästen 3.1, 3.2 ungefähr in ihrer Mitte in Querrichtung x gesehen Durchbrechungen 7 auf, die Durchsatz sowie inneren Teilkreislauf in z-Richtung ermöglichen. Die Hauptachse der Durchbrechungen 7 verläuft in Längsrichtung y (siehe Fig. 2A).

Der zur IR-Messung angeordnete Strahlungsaufbau 11 ist in frei durchstrahlbaren Querschnitten mit Volumenelementen wesentlicher Gas/Dampfkonzentration vorgesehen.

Die Gas/Dampfkonzentration ist trotz des "Rührkesselverhaltens" des Trockners aufgrund starker Strömungsdurchmischung nicht gleichmäßig verteilt. Partielle maximale Konzentrationswerte bestehen neben den Abdampfquellen, also neben der Bahn 1, im Falle der Verbrennung neben dem Brenner 16. Die Konzentration folgt in Längsrichtung y einer Kurve, die durch die zunehmende Erwärmung der Bahn nach ihrem Eintritt in den Eintrittsschlitz des Trockners ansteigt. Mit der Erwärmung, jedoch auch mit dem Dampftransport zur Grenzfläche des Papiers und der Druckfarbe steigt damit auch die Abdampfrate. Durchschnittliche Konzentrationswerte erscheinen an Strömungsstellen ohne Gas/Dampfquelle z. B. vor, hinter der Umluftströmungseinrichtung 4 und im Abgaskamin 8. Leckluft an Bahneintritts- und Austrittsschlitz 9, 10 spiegelt ebenfalls den Konzentrationsgang wieder.

Die Durchstrahlungsanordnung 11 mit Strahlungsquelle 11.1, Empfänger bzw. Detektor 11.2 durchmißt den Trockner in beliebiger Richtung.

Fig. 2B zeigt eine Strahlungsanordnung 11 mit y-Hauptstrahlrichtung. Dabei werden die in den im Strahlweg liegenden Volumenelementen erscheinenden, partiellen Konzentrationswerte integriert von der Koordinate y1 der Strahlungsquelle 11.1 bis zur Koordinate y2 des Empfängers 11.2.

Eine Überlagerung der IR-Absorption in y-Richtung mit der in x-Richtung, also in Bahnlängs- und -querrichtung entsteht bei Anordnung eines Ablenkspiegels 11.3, von dem der abgelenkte x-Quernebenstrahl von einem Reflektorspiegel 11.4 in den y-Strahlweg über den Ablenkspiegel 11.3 zurückkehrt.

Die Strahlungsanordnung 11 nach Fig. 2B zeigt prinzipiell folgende Einzelheiten:
Das von der Strahlungsquelle 11.1 ausgehende Strahlungsbüschel wird durch den sphärischen Spiegel 11.5 paraboloider oder ellipsoider Gestalt in den Meßstrahlweg parallel gerichtet. Die Konzentrierung des Parallelstrahls auf den Empfänger 11.2 erfolgt wiederum mit einem konzentrierenden, sphärischen Spiegel 11.5. Ein in den Meßstrahlweg gelegter Reflexionsspiegel, zweckmäßig ein Retroreflektor 11.6, reflektiert den Meßstrahl auf die Senderseite zurück, so daß Strahlungsquelle 11.1 und Empfänger 11.2 nebeneinander oder konzentrisch vorgesehen sind. Baulich bedingt die konzentrische Anordnung eine bestimmte Objektivgestalt.

Der in den Meßstrahlweg einbringbare Ablenkspiegel 11.3 wird bei einem weiterzuführenden Strahlwegteil als halbdurchlässiger Spiegel 11.7 ausgeführt, durch den ein Teil des Meßstrahls hindurchgeht und ein Teil den in Reflexionsrichtung liegenden Strahlweg des x-Quernebenstrahls einschlägt, von wo aus er durch den Reflektorspiegel 11.4 auf den halbdurchlässigen Spiegel 11.7 zurückfällt. Der Fangspiegel 11.8 spiegelt Nutzstrahlung zurück. Gleichzeitig zeigt die in Fig. 2A eingezeichnete Position des Fangspiegels 11.8, daß der Ablenkspiegel 11.3 von seiner aktiven, ablenkenden Spiegelposition in eine inaktive Position schwenkbar ist, womit der in x-Richtung verlaufende Strahlweg inaktiv würde und keine in x-Querrichtung der Bahn 1 vorhandenen Konzentrationen zur Schwächung beitragen würden.

Für die einwandfreie Funktion der optischen Elemente, die durch Kondensate verschmutzen können, sind den optischen Elementen apparativ jeweils kleine Heizteile zuordenbar, die der Kondensatbildung abhelfen. Ihre Einstelltemperatur liegt zweckmäßig über der maximalen Trocknerbetriebstemperatur.

Gegen optische Verschmutzung mit nichtsystematischem Meßfehlereffekt können auch Reinigungsteile vorgesehen werden.

Der Meßstrahlweg nach der Strahlungsanordnung 11 verläuft in y-Richtung parallel zur Bahn 1 oder Bahnkante. Längs der Durchbrechung 7 ist der Meßstrahlweg unbeeinflußt von Bahnschwingungen. Der Meßstrahl quer in x-Richtung verläuft parallel zur Kante der Düsenkästen 3.1, 3.2 oder zwischen den mit den Ausströmöffnungen versehenen Düsenleisten der Düsenkästen 3.1, 3.2.

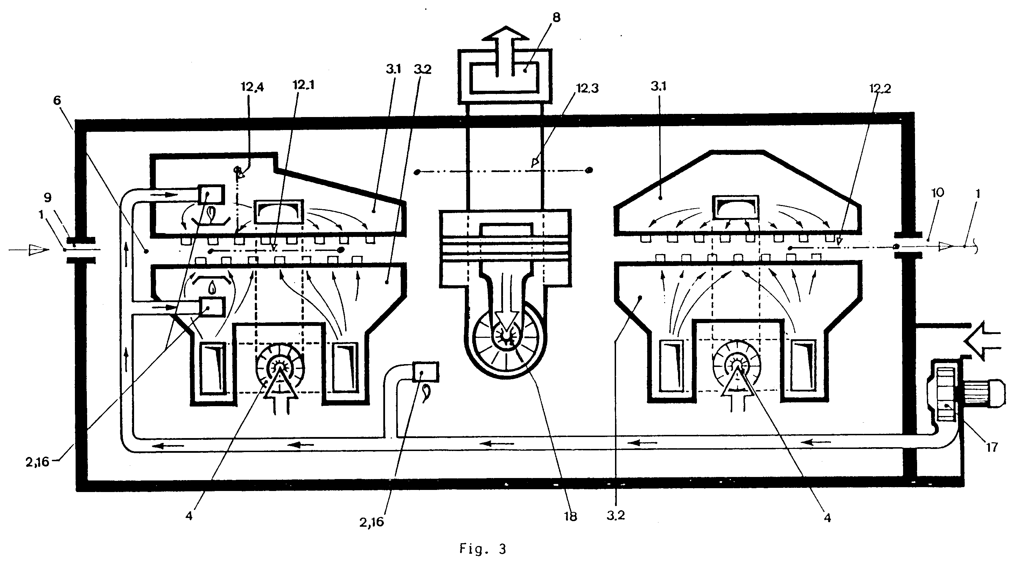

Fig. 3 zeigt einen Gasflammentrockner mit einem bahnaufwärtigem Paar von Düsenkästen 3.1, 3.2 und bahnabwärtigem Paar von Düsenkästen 3.1, 3.2. Die Bahn 1 wird dabei zur Trocknung zweifach beströmt. Zwischen den beiden Sektionen wird mit Dämpfen angereicherte Luft über den Abgaskamin 8 abgeführt.

Aufschluß über die Bildung der Druckfarbendämpfe und der Reinigungsflüssigkeitsdämpfe ist möglich, wenn die Konzentrationsverteilung an verschiedenen Stellen über verschiedene Meßstrecken gemessen wird. Dazu ist eine erste Meßstrecke 12.1 in Höhe des ersten Paars der Düsenkösten 3.1, 3.2 eingerichtet. Eine zweite Meßstrecke 12.2 bezieht sich auf das zweite Paar der Düsenkästen 3.1, 3.2. Die Meßstrecken 12.1, 12.2 sind dadurch apparativ miteinander zu koppeln, indem in einem sich über den Trockner erstreckenden Strahlengang Spiegel z. B. als Retroreflektoren (Katzenaugen) eingeschwenkt oder eingeschoben werden, wodurch kurze oder längere Durchstrahlungsabschnitte erzeugbar sind.

Das Prinzip der Strahlwegverzweigung mit evtl. nur einem Sender 11.1 und einem Empfänger 11.2 durch Aufspaltung in Haupt- und Nebenstrahlweg ist in Fig. 2B angegeben.

Die Meßstrecke 12.3 dient der Aufnahme der Durchlässigkeit des Abgases, in dem sich summarisch alle Gase/Dämpfe vereinigen. Nachdem im Trockner zirkulierende Gasteilvolumina kürzer oder länger verweilen, ist die durchschnittliche Verweilzeit der im Abgaskamin vorfindbaren Gasanteile am höchsten. Die Meßstrecke 12.3 bietet damit nicht die Messung schnell entstehender Gase/Dämpfe. Sie ist jedoch vom kompaktem Meßaufbau her und von der Bilanzierungsmöglichkeit der Gas/Dampfanteile her besonders geeignet.

Die Meßstrecke 12.4 ist in die Strömung vor die ungeschützte Heizung, hier Brenner 16, gelengt. Die Strömung dort sollte über vorzusehende Zusatzhilfsmittel kontrolliert, reproduzierbar verlaufen.

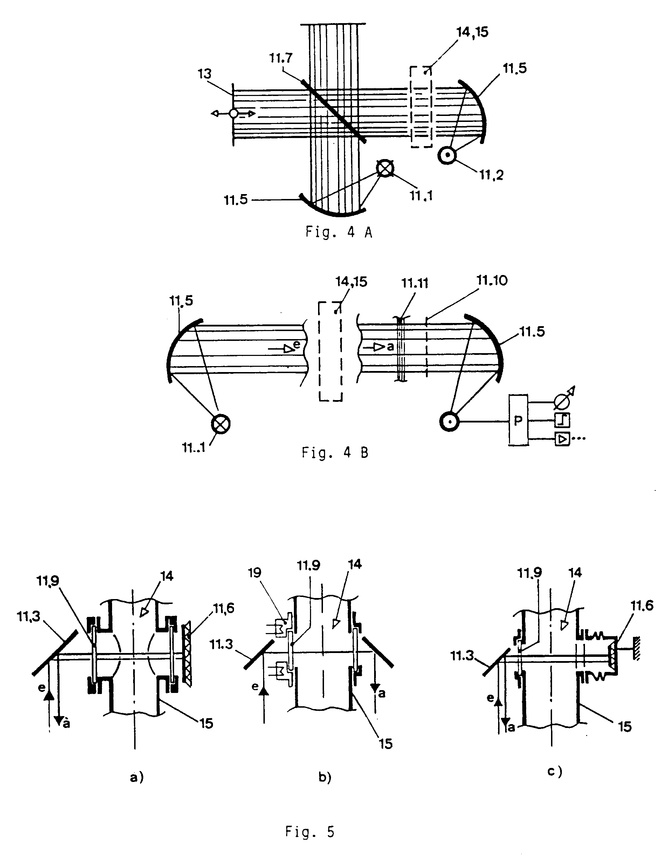

Bei Anordnung eines nach der dispersiven Methode arbeitenden, ein Interferometer enthaltenden FTIR-Spektrometers werden alle absorptionsbehafteten Anteile abgesehen von unvermeidbaren Bandenüberschneidungen diskriminiert. In Fig. 4A ist ein solches FTIR-Spektrometer prinzipiell dargestellt. In einem Geräteteil des Spektrometers befindet sich ein Spiegel 13 mit Vortrieb. Ein Computer verarbeitet die an- und abschwellende Strahlungsstärke der Interferenzmaxima in Abhängigkeit vom Vortrieb des Spiegels 13. Die Strahlungsstärke wird nach Fourier zerlegt. Aufgrund des über den ausmeßbaren IR-Bereich zustande kommenden Spektrums liegen alle absorbierenden Gas/Dampfanteile mehr oder weniger voneinander diskriminierbar vor. Das aus den Figuren 4A und 4B ersichtliche Probenteil 14 entspricht den Meßstrecken 12.1 bis 12.4. Das Eingangsstrahlenbündel e trifft auf das Probenteil 14, wo die spezifische Absorption stattfindet und gelangt als Ausgangsstrahlenbündel a zum Empfänger 11.2.

Bei dem spektrometrischen Aufbau nicht dispersiver Art nach Fig. 4B wird auf bestimmte Wellenlängen abgestellt, indem der Empfänger 11.2 selektiv geartet ist oder ein Filter 11.10 zur Selektion im Strahlengang angeordnet ist. Zu Kalibrier-, Vergleichs- und Meßzwecken ist ein Zielgas, entweder das abgezielte Meßgas wie z. B. ein wesentlicher Kohlenwasserstoffdampf der Waschflüssigkeit oder ein Tracer-Gas ebenfalls in den Strahlengang der Strahlungsanordnung 11 schaltbar, siehe filterartiger Vergleichsstoff 11.11.

Das Ausgangssignal des Empfängers 11.2 nach Fig. 4B führt zu einem Prozessor P, der die Auswertung vornimmt. Ihm nachgeordnet sind Anzeige, Grenzwertmelder, Verstärkerleitungen zu den Stellgliedern des Trockners, der Waschbalken und zu Not-Aus der Druckmaschine. Der Rechner ist mit dem Managementsystem gekoppelt, wo auch die Druckfarbenauswertung anhand der Bindemittel-Kohlenwasserstoffdämpfe ausgegeben wird.

Die Figuren 5A, B, C zeigen die Anwendung der Strahlengang-Gestaltung mit Strahlenbünden e, a auf ein apparativ begrenztes Volumenelement des Trockners, das sich zwischen den Wänden eines Rohrs oder sonstigen Behältnis- oder Leitungsteils 15 befindet. Nach Fig. 5A verlaufen die Strahlenbündel e, a durch angeflanschte Fenster 11.9 über einen Retroreflektor 11.6, der das Strahlenbündel a auf die Senderseite zurücklenkt, wodurch die Strahlungsquelle 11.1 und der Strahlungsempfänger 11.2, also Sende- und Empfangsteil, in einem Gerät zusammenlegbar sind. Nach Fig. 5B gibt die optische Bank einen Strahlengang diesseits und jenseits des Apparateteils 15. Die auf jeder Seite des Apparateteils 15 angeordneten Ablenkspiegel 11.3 kommen mit den Gasen/Dämpfen nicht in Berührung.

Ein Blockflansch des Fensters 11.9 ist mit einem Heizteil 19 versehen.

Fig. 5C zeigt eine Variante zur Fig. 5A mit ortsfest montiertem Retroreflektor 11.6. Diese Anordnung verhindert, daß der Retroreflektor 11.6 aufgrund der Erwärmung und Spannung des Apparateteils 15 auswandert. Die optische Bank bleibt somit stabil.

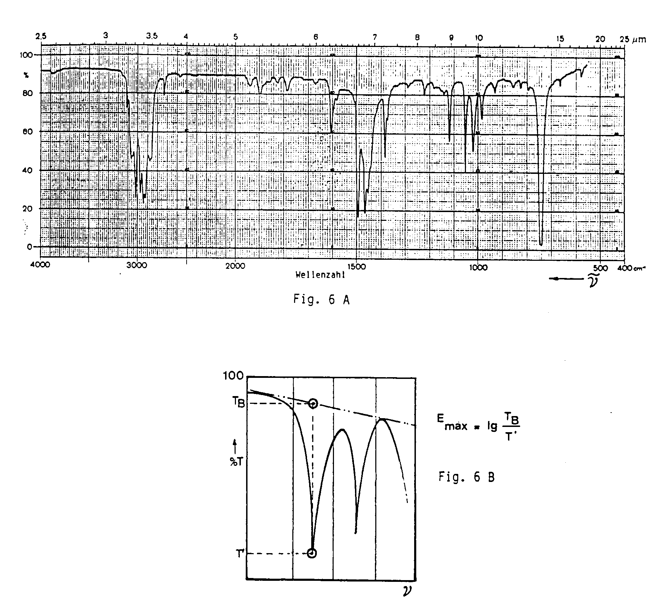

Unabhängig davon, ob spezifische Banden aus einem aufgenommenem Spektrum, siehe Fig. 6A, oder einem gefilterten schmalbandigen Bereich des Spektrums untersucht und ausgewertet werden, ist die jeweilige ,Absorptionsverlust zeigende Schlüsselbande mit einer Intensität ungefähr konstanten Werts zu vergleichen. Günstig ist der Bezug der fraglichen Bandenintensität auf eine klar festzustellende, isoliert liegende Absorptionsbande jeweils realtiv konstanten Werts. Eine Bezugsintensität ist auch durch eine Durchlässigkeitsmaxima verbindende Basislinie festlegbar. Die zur Gas/Dampfkonzentration proportionale Extinktion ergibt sich näherungsweise nach dem auf Basislinienbildung beruhenden Aus wertverfahren durch den logarithmierten Quotienten T _{B}/T′. Auch ohne numerische Auswertung zeigt die Bandenintensität sofort, ob im Trocknungsprozeß anwachsende oder abnehmende Gas/Dampfkonzentration abläuft.

Aus dem Absorptionsspektrum sind manuell, mathematisch (siehe Fourier-Analyse) oder optisch selektiv alle IR-nachweisbaren Stoffe bzw. Substanzen nachweisbar. So zeigt die relativ eindeutig feststellbare CO-Bande den Grad der Verbrennung im Brenner 16 an. Weitere Nachweisbanden des Brenngases zeigen, inwieweit die Verbrennung vollständig ist.

Während die FID-Meßmethode lediglich zum Gesamtkohlenwasserstoff führt und hierbei auch die Kohlenoxide subsummiert sind, ist bei dem erfindungsgemäßen IR-Meßaufbau im Trockner eine Trennung in die auf die Verbrennung, die Druckfarbenverdampfung und die Reinigungsflüssigkeitsverdampfung entfallenden Stoffe möglich.

Mit Abhängigkeit der in der Trockneratmosphäre bzw. Gas/Dampfphase vorfindbaren Konzentrationen von den Einstellwerten der Betriebsparameter des Trockners können die gemessenen Bandenintensitäten roh oder ausgewertet den Regeleinrichtungen des Trockners aufgeschaltet werden. Da prozeßtechnisch zu verändernde Konzentrationswerte zwangsläufig Eingriff auf die Stoffdosierung - bei der Farbe auf die Farbführung und bei dem Gummituchwaschen auf die Reinigungsflüssigkeitszuführung des Waschbalkens - bedingen, wird auch von der gemessenen Bandenintensität ein Führungssignal für die jeweilige Dosierung abgeleitet. Außer sofortiger rückgehender Dosierung bei festgestellter Überkonzentrierung vor allem explosionsgefährlicher Dämpfe ist gleichermaßen Verdünnung durch höheren Zuluft- und Umluftstrom sowie Temperaturabsenkung möglich.

Aus aufgenommenen Bandenintensitäten und aufgenommenen Einstellwerten der Druckprozeß- und Trocknerstelleinrichtungen sind Voreinstellwerte für den Folgebetrieb mit z. B. Wiederholaufträgen gewinnbar.

## Patentansprüche

1. Durchlauftrockner von Rollenrotationsdruckmaschinen zur Trocknung von bedruckten Stoffbahnen (1),
- mit einer Trockenzone zur Beaufschlagung der in einem Bahnkanal (6) geführten Bahn (1) mit einem erhitzten Hilfsgas, vorzugsweise Luftstrom (5),
- mit Regler-anschließbaren Stelleinrichtungen für Heizung, Strömung, Lüftung einschließlich zugehörigen Meßaufnehmern im Trockner für den sich aus Zuluft, Umlaufluft und Abluft zusammensetzenden Luftstrom (5),
- mit Stelleinrichtungen außerhalb des Trockners für die Farbmittelführung, Feuchtmittelführung sowie Bahngeschwindigkeit und Reinigungsflüssigkeitszufuhr,
- mit mindestens einer auf eine obere festgelegte, von Kohlenwasserstoffen kommende Gas/Dampfkonzentration kalibrierten Gaswarneinrichtung,
**dadurch gekennzeichnet,**
daß im Luftstrom (5) ein sich zwischen einer IR-Strahlenquelle (11.1) und einem IR-Empfänger (11.2) erstreckender Strahlengang (11) mit optischen Elementen (11.1 bis 11.11) als Meßstrecke (12.1 bis 12.4) angeordnet ist, daß eine Meß- und Auswerteschaltung vorgesehen ist, welche aus einem empfangenen Intensitätsverlauf, bestimmten Gas/Dampfkomponenten zuordenbare, spezifische Banden oder Bandensätze hinsichtlich ihrer Intensität quantifiziert, welche außerdem die Bandenintensitätswerte gemäß einer Methode der quantitativen Auswertung wie z.B. dem Grundlinienverfahren normiert und somit vergleichsfähige relative Durchlässigkeitswerte oder Extinktionswerte erzeugt, und welche fortlaufend online-gemessene Intensitäten der spezifischen Banden mit einem gewählten, vorgebbaren Extinktionswert vergleicht, daß eine Regeleinrichtung mit einem vorgebbaren Führungsverlauf der Extinktion entsprechend der Gas/Dampfkonzentration der spezifischen Gase/Dämpfe vorgesehen ist, welche durch Soll-Ist-Vergleich zwischen vorliegender (Ist-Wert) und zu führender Gas/Dampfkonzentration (Soll-Wert) die Stelleinrichtungen betätigt.

2. Betriebsverfahren eines Durchlauftrockners einer Rollenrotationsdruckmaschine gemäß Anspruch 1 bei Fortdruck und beim Zylinderwaschen mit laufender Bahn,
**dadurch gekennzeichnet,**
daß die Bandenintensitäten der spezifischen Gase online kontinuierlich während des Fortdrucks und während des Zylinder- oder Gummituchwaschens quantifiziert werden, daß Ist-Werte der Extinktion entsprechend der Gas/Dampfkonzentration durch Verstellung der Stellglieder hin zu einem Extinktions- bzw. Konzentrations-Führungsgrößenwert geändert werden, wobei der Führungsgrößenwert kleiner als ein oberer Grenzwert ist, daß ein Führungsgrößenverlauf bezüglich des Fortdrucks auf der Extinktion entsprechend der Gas/Dampfkonzentration mindestens einer der Flüssigkomponenten (Bindemittel) der Druckfarbe beruht, daß ein anderer Führungsgrößenverlauf bezüglich des Gummituchwaschens auf der Extinktion entsprechend der Gas/Dampfkonzentration mindestens einer der Waschmittelkomponenten, insbesondere Waschbenzin, beruht und daß die Regeleinrichtung für den einen und den anderen Führungsgrößenverlauf ausgebildet ist.

3. Betriebsverfahren eines Durchlauftrockners einer Rollenrotationsdruckmaschine gemäß Anspruch 1, bei dem die Aufheizung der Trocknungsatmosphäre im Trockner durch offene Verbrennung eines Brenngases in einem Brenner (16) erzeugt wird,
**dadurch gekennzeichnet,**
daß die Intensitäten der spezifischen Banden oder Bandensätze mindestens einer Komponente (z.B. CH₄) des Brenngases und/oder mindestens einer Komponente des Abgases (z.B. CO) quantifiziert wird, daß die Intensitäten als relative Durchlässigkeit oder Extinktion nach einem bekannten quantitativen Auswerteverfahren dargestellt werden, daß die Verbrennung in Abhängigkeit der Einstellung des Brenners (16) sowie Temperatur in Verbindung mit den Einstellwerten der Stelleinrichtungen des Trockners anhand der Extinktionswerte mit dem Ziel der vollkommenen Verbrennung, d.h. ohne Brenngasreste und ohne CO, kontrolliert wird, wobei durch die die zugeführten Extinktionswerte verarbeitende Regeleinrichtung die Stelleinrichtungen des Brenners (16) betätigt werden.

4. Betriebsverfahren eines Durchlauftrockners einer Rollenrotationsdruckmaschine gemäß Anspruch 1 bei Fortdruck,
**dadurch gekennzeichnet,**
daß mittels der IR-Meßstrecken (12.1 bis 12.4) die Intensitäten der Banden oder Bandensätze von Flüssigkeitskomponenten (Bindemittel) der Druckfarbe on-line-quantifiziert werden, daß die Intensitäten als relative Durchlässigkeit oder Extinktion auf der Grundlage eines bekannten Auswerteverfahrens ausgedrückt werden, daß die kontinuierlich gemessenen Bandenintensitäten oder Extinktionswerte mit vorgegebenen oder gespeicherten Werten verglichen werden, daß Abweichungen der den Extinktionswerten entsprechenden Konzentrationswerte der Bindemittelsubstanzen gegenüber den Vergleichswerten festgestellt und registriert werden, daß die Trocknerführung an die andere Farbkomponentenverdampfung anhand Stellwertveränderung der Stelleinrichtungen des Brenners (16), Klappen und Drehzahl der Strömung-/Lüftungseinrichtungen (4, 17, 18) durch die Regeleinrichtung angepaßt wird.

5. Durchlauftrockner nach Anspruch 1,
**dadurch gekennzeichnet,**
daß der Strahlengang (11) in einem der Strömungswege des Luftstroms (5) angeordnet ist, wobei die Anordnung wahlweise vorgesehen ist:
- längs der Bahn (1) in y-Richtung (Meßstrecke 12.1, 12.2, Durchbrechung 7),
- quer zur Bahn (1) in x-Richtung (Meßstrecke 12.1, 12.2),
- der Heizungseinrichtung (2, 16) direkt vor- oder nachgeordnet (Meßstrecke 12.4),
- dem Abluftstrom zugeordnet (Meßstrecke 12.3),
- im Umluftstrom.

6. Durchlauftrockner nach Anspruch 1 oder 5,
**dadurch gekennzeichnet,**
daß die Meßstrecke (12.1, 12.2) in einem Leerraum in x-Richtung zwischen Düsenleisten von Düsenkästen (3.1, 3.2) angeordnet ist.

7. Durchlauftrockner nach Anspruch 1, 5 oder 6,
**dadurch gekennzeichnet,**
daß im Trockner befindliche optische Elemente (11.1 bis 11.11) über Trocknerinnentemperaturen durch Heizteile (19) heizbar sind.

8. Durchlauftrockner nach Anspruch 1 oder einem der Ansprüche 5 bis 7,
**dadurch gekennzeichnet,**
daß im Strahlengang (11) angeordnete Reflektorspiegel (11.4) als Retroreflektoren (11.6) ausgebildet sind, daß für Strahlteilung halbdurchlässige Spiegel (11.7) angeordnet sind, und daß die optischen Elemente (11.1-11.11) schwenk-, verschieb-, abdeckbar sind.

9. Durchlauftrockner nach Anspruch 1 oder einem der Ansprüche 5 bis 8,
**dadurch gekennzeichnet,**
daß Filter (11.10), Vergleichsstoff (11.11) oder Empfänger (11.2) zur selektiven Trennung und Auswertung der spezifischen Kennbanden eines IR-Spektrums (z.B. 4000 bis 400 cm⁻¹) vorgesehen sind, wobei der Empfänger (11.2) matrixartig eine Mehrzahl von bei verschiedenen Wellenlängen selektierenden Elementen aufweisen kann.

10. Betriebsverfahren nach einem der Ansprüche 2 bis 4 für einen Durchlauftrockner nach einem der Ansprüche 1 oder 5 bis 9,
**dadurch gekennzeichnet,**
daß die auf die Stelleinrichtungen wirkende Regeleinrichtung beim Übergang von Fortdruck auf Gummituchwaschen von den der Regeleinrichtung zugrundeliegenden Einstellparametern für Fortdruck auf andere Regelparameter für Gummituchwaschen umgeschaltet wird.

## Claims

1. A continuous-flow drier for web-fed rotary presses for drying printed webs of fabric (1),
- having a dry zone for supplying the web (1) guided in a web channel (6) with a heated auxiliary gas, preferably air stream (5),
- having actuating devices, which can be connected to the control unit, for heating, flow, ventilation including associated measurement recorders in the drier for the air stream (5) composed of incoming air, circulating air and outgoing air,
- having actuating devices outside the drier for the control of colouring agents, moistening agents and also web velocity and supply of cleaning fluid,
- having at least one gas detector calibrated to an upper fixed gas/vapour concentration coming from hydrocarbons,
**characterised in that** a beam path (11) extending between an IR radiation source (11.1) and an IR sensor (11.2) and having optical elements (11.1 to 11.11) is disposed in the air stream (5) as a measured length (12.1 to 12.4), **in that** a measurement and evaluation circuit is provided, which from a detected intensity pattern quantifies specific bands or band sets, which can be allocated to determined gas/vapour constituents, with respect to their intensity, which also scales the band intensity values according to a method of quantitative evaluation such as, for example, the base line method, and thus produces relative transparency values or extinction values suitable for comparison, and which continuously compares on-line measured intensities of the specific bands with a selected, predetermined extinction value,
**in that** a control mechanism having a predetermined control pattern of the extinction according to the gas/vapour concentration of the specified gases/vapours is provided, which actuates the actuating devices by a variance comparison between the available gas/vapour concentration (actual value) and the gas/vapour concentration to be controlled (desired value).

2. An operating process for a continuous-flow drier in a web-fed rotary press as specified in Claim 1 during a control run and during the washing of the cylinder with the moving web,
**characterised in that** the band intensities of the specific gases are continuously quantified on-line during the production run and during the washing of the cylinder or rubber blanket,
in that actual values of the extinction according to the gas/vapour concentration are altered by adjusting the actuating elements to an extinction or concentration command variable value, whereby the command variable value is less than an upper limit value,
**in that** a command variable pattern with respect to the production run is based on the extinction according to the gas/vapour concentration of at least one of the fluid constituents (binder) of the printing ink,
**in that** another command variable pattern with respect to the washing of the rubber blanket is based on the extinction according to the gas/vapour concentration of at least one of the washing medium constituents, in particular petroleum ether,
**and in that** the control mechanism is designed for both command variable patterns.

3. An operating process for a continuous-flow drier in a web-fed rotary press as specified in Claim 1, in which the heating of the drying atmosphere in the drier is produced by open combustion of a fuel gas in a burner (16),
**characterised in that** the intensities of the specific bands or band sets of at least one constituent (e.g. CH₄) of the fuel gas and/or of at least one constituent of the exhaust gas (e.g. CO) are quantified,
**in that** the intensities are represented as relative transparency or extinction according to a known quantitative evaluation method,
**in that** combustion is controlled in dependence on the setting of the burner (16) and also the temperature is controlled in conjunction with the set values of the actuating devices of the drier by means of the extinction values with the aim of complete combustion, i.e. without fuel gas residues and without CO, whereby the actuating devices of the burner (16) are actuated by the control mechanism processing the supplied extinction values.

4. An operating process for a continuous-flow drier in a web-fed rotary press as specified in Claim 1 in a production run,
**characterised in that** the intensities of the bands or sets of bands of fluid constituents (binder) of the printing ink are on-line quantified by means of the IR measured lengths (12.1 to 12.4),
**in that** the intensities are expressed as the relative transparency or extinction on the basis of a known evaluation method,
**in that** the continuously measured band intensities or extinction values are compared with predetermined or stored values,
**in that** differences between the concentration values of the binder substances corresponding to the extinction values and comparative values are determined and registered,
**in that** the drier control is adapted to the evaporation of the other ink constituent by altering the manipulated value of the actuating devices of the burner (16), valves and speed of the flow/ventilation devices (4, 17, 18) by the control mechanism.

5. A continuous-flow drier according to Claim 1,
**characterised in that** the beam path (11) is disposed in one of the flow paths of the air stream (5), whereby the arrangement is provided according to choice:
- along the web (1) in the y-direction (measured length 12.1, 12.2, opening 7),
- at right angles to the web (1) in the x-direction (measured length 12.1, 12.2),
- directly in front of or behind the heating installation (2, 16) (measured length 12.4),
- associated with the outgoing air stream (measured length 12.3),
- in the circulating air stream.

6. A continuous flow drier according to Claim 1 or 5,
**characterised in that** the measured length (12.1, 12.2) is disposed in an empty space in the x-direction between nozzle lips of nozzle casings (3.1, 3.2).

7. A continuous-flow drier according to Claim 1, 5 or 6,
**characterised in that** optical elements located in the drier (11.1 to 11.11) can be heated above temperatures prevailing inside the drier by heating parts (19).

8. A continuous-flow drier according to Claim 1 or one of Claims 5 to 7,
**characterised in that** reflector mirrors (11.4) disposed in the beam path (11) are constructed as retroreflectors (11.6),
**in that** semi-transparent mirrors (11.7) are provided for the beam splitting,
**and in that** the optical elements (11.1 - 11.11) can be swivelled, displaced and uncovered.

9. A continuous-flow drier according to Claim 1 or one of Claims 5 to 8,
**characterised in that** filter (11.10), comparison substance (11.11) or sensor (11.2) are provided for the selective separation and evaluation of the specific identification bands of an IR spectrum (e.g. 4000 to 400 cm⁻¹), whereby, like a matrix, the sensor (11.2) may comprise a plurality of elements performing a selection at various wavelengths.

10. An operating process according to one of Claims 2 to 4 for a continuous-flow drier as specified by one of Claims 1 or 5 to 9,
**characterised in that**, with the change from a production run to rubber blanket washing, the control mechanism acting on the actuating devices is switched over from the control parameters for a production run on which the control mechanism is based to other control parameters for washing the rubber blanket.

## Revendications

1. Sécheur continu de rotatives d'imprimerie à bobines, destiné au séchage de bandes imprimées (1), comprenant
- une zone de séchage pour appliquer à la bande (1), guidée dans un canal de bande (6), un gaz auxiliaire échauffé, de préférence un courant d'air (5),
- des dispositifs d'asservissement ou de positionnement, pouvant être raccordés à un régulateur, pour le chauffage, la circulation d'air, la ventilation, y compris des capteurs associés dans le sécheur pour le courant d'air (5), composé d'air d'arrivée, d'air recyclé et d'air évacué,
- des dispositifs d'asservissement ou de positionnement à l'extérieur du sécheur pour l'application d'encre, l'application de produit de mouillage ainsi que la vitesse de défilement de la bande et l'amenée de liquide de nettoyage, de même que
- au moins un dispositif détecteur de gaz calibré sur une concentration supérieure déterminée de gaz/vapeurs provenant d'hydrocarbures,
caractérisé en ce que
une marche de rayons (11), s'étendant entre une source de rayonnement infrarouge (11.1) et un récepteur infrarouge (11.2) et comprenant des éléments optiques (11.1 à 11.11), est disposée en tant que parcours de mesure (12.1 à 12.4) dans le courant d'air (5), qu'un circuit de mesure et d'exploitation est prévu, circuit qui, à partir d'une allure d'intensité reçue, quantifie, pour ce qui concerne leur intensité, des bandes ou des séries de bandes spécifiques pouvant être coordonnées à des composants déterminés des gaz/vapeurs, qui normalise en outre les valeurs d'intensité des bandes selon une méthode de l'évaluation quantitative, comme par exemple le procédé à ligne de base, et génère ainsi des valeurs de transmission ou des valeurs d'extinction relatives se prêtant à une comparaison, et qui compare en continu des intensités mesurées en direct des bandes spécifiques avec une valeur d'extinction choisie, pouvant être fixée d'avance, et qu'un dispositif de réglage est prévu avec une allure de référence de l'extinction pouvant être préfixée en conformité avec la concentration en gaz/vapeur des gaz/vapeurs spécifiques, dispositif de réglage qui, par une comparaison consigne-réel entre la concentration des gaz/vapeurs actuelle (valeur réelle) et la concentration des gaz/vapeurs à obtenir (valeur de consigne), actionne les dispositifs d'asservissement.

2. Procédé d'exploitation d'un sécheur continu d'une rotative d'imprimerie à bobines selon la revendication 1 pendant le tirage continu et pendant le lavage des cylindres alors que la bande défile,
caractérisé en ce que
les intensités de bandes des gaz spécifiques sont quantifiées de façon continue, en direct, pendant le tirage continu et pendant le lavage des cylindres ou des blanchets, que des valeurs réelles de l'extinction sont changées en conformité avec la concentration des gaz/vapeurs par l'ajustement des organes d'asservissement vers une valeur de grandeur de référence d'extinction ou de concentration, la valeur de grandeur de référence étant plus petite qu'une valeur limite supérieure, qu'une allure de grandeur de référence relative au tirage continu est basée sur l'extinction en conformité avec la concentration en gaz/vapeur d'au moins un des composants liquides (liant) de l'encre d'imprimerie, qu'une autre allure de grandeur de référence, relative au lavage des blanchets, est basée sur l'extinction en conformité de la concentration en gaz/vapeur d'au moins un des composants du produit de lavage, en particulier d'éther de pétrole, et que le dispositif de réglage est conçu pour l'une ou l'autre allure de grandeur de référence.

3. Procédé d'exploitation d'un sécheur continu d'une rotative d'imprimerie à bobines selon la revendication 1, dans lequel l'échauffement de l'atmosphère de séchage dans le sécheur est produit par combustion ouverte d'un gaz combustible dans un brûleur (16),
caractérise en ce que
les intensités des bandes ou des séries de bandes spécifiques d'au moins un composant (par exemple CH₄) du gaz combustible et/ou au moins un composant du gaz évacué (par exemple CO) sont quantifiées, que les intensités sont représentées, selon un procédé connu d'évaluation quantitative, comme une transmission relative ou une extinction relative, et que la combustion est contrôlée, en fonction du réglage du brûleur (16), ainsi que de la température, en liaison avec les valeurs d'ajustement des dispositifs d'asservissement du sécheur, d'après les valeurs d'extinction, avec le but d'obtenir la combustion complète, c'est-à-dire sans résidus du gaz combustible et sans CO, les dispositifs d'asservissement du brûleur (16) étant actionnés par le dispositif de réglage, traitant les valeurs d'extinction qui lui sont amenées.

4. Procédé d'exploitation d'un sécheur continu d'une rotative d'imprimerie à bobines selon la revendication 1 lors du tirage continu,
caractérisé en ce que
les intensités des bandes ou des séries de bandes de composants liquides (liant) de l'encre d'imprimerie sont quantifiées en direct au moyen des parcours de mesure infrarouge (12.1 à 12.4), que les intensités sont imprimées en sortie sous la forme d'une transmission relative ou d'une extinction relative, sur la base d'un procédé d'évaluation connu, que les intensités de bandes ou les valeurs d'extinction, mesurées en continu, sont comparées avec des valeurs préfixées ou mémorisées, que des écarts des valeurs de concentration correspondant aux valeurs d'extinction des substances de liant, par rapport aux valeurs de comparaison, sont constatés et enregistrés et que la conduite du sécheur est adaptée par le dispositif de réglage à l'autre évaporation de composants d'encre à l'aide d'une modification de la valeur d'asservissement des dispositifs d'asservissement du brûleur (16), de volets et de la vitesse de rotation des dispositifs de circulation et de ventilation d'air (4, 17, 18).

5. Sécheur continu selon la revendication 1, caractérisé en ce que la marche de rayons (11) est disposée dans un des trajets d'écoulement du courant d'air (5), le dispositif étant prévu, au choix:
- le long de la bande imprimée (1) dans la direction y (parcours de mesure 12.1, 12.2, interruption 7),
- transversalement à la bande (1) dans la direction x (parcours de mesure 12.1, 12.2),
- directement devant ou derrière le dispositif de chauffage (2, 16) (parcours de mesure 12, 4),
- en coordination avec le courant d'air évacué (parcours de mesure 12.3) ou
- dans le courant d'air recyclé.

6. Sécheur continu selon la revendication 1 ou 5, caractérisé en ce que le parcours de mesure (12.1, 12.2) est disposé dans un espace vide, suivant la direction x, entre des rampes de buses de caissons à buses (3.1, 3.2).

7. Sécheur continu selon la revendication 1, 5 ou 6, caractérisé en ce que des éléments optiques (11.1 à 11.11) se trouvant à l'intérieur du sécheur, peuvent être chauffés par des parties de chauffage (19) au-dessus des températures régnant à l'intérieur du sécheur.

8. Sécheur continu selon la revendication 1 ou une des revendications 5 à 7, caractérisé en ce que des miroirs réflecteurs (11.4) placés dans la marche de rayons (11) sont réalisés comme des rétroréflecteurs (11.6), que des miroirs semi-transparents (11.7) sont prévus pour la séparation du faisceau, et que les éléments optiques (11.1 - 11.11) sont mobiles en pivotement ou en translation et peuvent être recouverts.

9. Sécheur continu selon la revendication 1 ou une des revendications 5 à 8, caractérisé en ce que des filtres (11.10), une matière de comparaison (11.11) ou un récepteur (11.2) sont prévus pour la séparation et l'évaluation sélectives des bandes d'identification spécifiques d'un spectre infrarouge (allant par exemple de 4000 à 400 cm⁻¹), le récepteur (11.2) pouvant présenter, à la façon d'une matrice, une pluralité d'éléments agissant sélectivement à différentes longueurs d'onde.

10. Procédé d'exploitation selon une des revendications 2 à 4 pour un sécheur continu selon une des revendications 1 ou 5 à 9, caractérisé en ce que le dispositif de réglage, agissant sur les dispositifs d'asservissement, est commuté, lors du passage du tirage continu au lavage des blanchets, pour passer des paramètres d'ajustement de base du dispositif de réglage pour le tirage continu, à d'autres paramètres de réglage, se rapportant au lavage des blanchets.
